# EUROPEAN PATENT APPLICATION

(11) **EP 2 492 665 A1**
(43) Date of publication of application: **29.08.2012**
(21) Application number: 10824893.1
(22) Date of filing: 18.10.2010
(51) Int. Cl.: G01N 21/59, G01N 21/17, G01N 33/543

(54) **BIOSENSOR USING MAGNETIC MICROPARTICLES**

(30) Priority: 19.10.2009 JP 2009240268
(71) Applicant: Tokyo Institute of Technology, Tokyo 152-8550 (JP); Sumitomo Electric Industries, Ltd., Chuo-ku Osaka-shi Osaka 541-0041 (JP)
(72) Inventor: SANDHU, Adarsh, Tokyo 152-88550 (JP)
(74) Representative: Skone James, Robert Edmund
(86) International application number: PCT/JP2010/068273
(87) International publication number: WO 2011/049044

(57) **Abstract**

Provided is a biosensor using magnetic microparticles whereby a biomaterial can be detected at a high sensitivity. The biosensor using magnetic microparticles is provided with a light source (10), a magnetic field generator (20), a light receiver (30) and a detector (40). The light source (10) irradiates a dispersion (1) containing the magnetic microparticles, to which the biomaterial to be detected can bind, with light of a definite wavelength. The magnetic field generator (20) applies to the dispersion a magnetic field capable of changing at least in two directions. The light receiver (30) receives transmitting light from the dispersion (1). The detector (40) detects the presence or absence of the target biomaterial based on the magnitude of a change in the quantity of the transmitting light, which changes in response to a change in the direction of the magnetic field applied by the magnetic field generator (20), received by the light receiver (30).

## Description

### Technical Field

The present invention relates to a biosensor using magnetic microparticles, and more particularly to a biosensor that applies a magnetic field to a dispersion liquid containing magnetic microparticles.

### Background Art

These days, biosensors are used in a wide range of fields, such as analysis of the interaction between proteins, cancer therapy, DNA analysis, detection of pathogens and the like, diagnosis of diseases, and measurement of environment-related materials. Biosensors are designed to carry out qualitative and quantitative analysis of a to-be-measured material by measuring the binding of an antigen (biomaterial), which is the to-be-measured material, and an antibody, which is a test reagent that binds specifically to the antigen.

As to a technique for measuring the binding of the antigen and the antibody, there is a DNA chip that for example uses a fluorescent material and carries out the measurement based on shading of colors. However, the light intensity of the fluorescent material or the like is unstable, and the DNA chip is therefore not suited for high-accuracy measurement.

There is also a biosensor using the measurement of mass change by utilizing a Surface Plasmon Resonance measurement method (SPR) or a Quartz Crystal Microbalance measurement method (QCM). However, it is not possible to obtain sensitivity sufficient enough to measure a biomaterial that is extremely small in mass. Moreover, devices become complicated. There are also cost and other problems.

As to a biosensor that has recently gained attention, the following and other methods are known: a method of using a magnetic microparticle, to which a biomaterial is fixed, as a marker, and using a magnetic sensor that uses a Giant Magneto- Resistive (GMR) element or Hall element to measure the magnetism of the magnetic microparticle.

The device that measures the magnetism of the magnetic microparticle may not be able to obtain sensitivity sufficient enough because the magnetism becomes extremely small as the diameter of the magnetic microparticle decreases.

Moreover, what is disclosed in Patent Document 1 is one that is able to detect a to-be-measured antigen or antibody by applying a magnetic force to a dispersion liquid containing magnetic microparticles in one direction, releasing the agglutinated magnetic microparticles after agglutinating forcibly, emitting light to the dispersion liquid at that time, receiving the scattered light, and using the amount of light received.

### Citation List

### Patent Document

Patent Document 1: Japanese Patent Application Kokai Publication No Hei 05-240859

### Disclosure of the Invention

### Problems to be Solved by the Invention

However, the one disclosed in Patent Document 1 is only designed to release after applying a magnetic force once in one direction, and then obtain the resultant turbidity just by measuring the transmitted light. Since precipitation occurs as time goes by, the turbidity also changes. Accordingly, it is not possible to accurately measure the antigen or antibody.

In view of the above circumstances, the object of the present invention is to provide a biosensor that uses a magnetic microparticle and is able to detect a biomaterial with higher levels of sensitivity.

### Means for Solving the Problems

To achieve the above object of the present invention, a biosensor of the present invention, which uses magnetic microparticles, comprises: a light source that emits light of a predetermined wavelength to a dispersion liquid that contains magnetic microparticles to which a target biomaterial can bind; a magnetic field generation unit that is able to apply a magnetic field, a direction of which changes at least in two directions, to the dispersion liquid; a light receiving unit that receives a transmitted light from the dispersion liquid; and a detection unit that detects whether or not the target biomaterial exists based on an amount of change in a quantity of the transmitted light received by the light receiving unit, which is caused by a direction change of the magnetic field applied by the magnetic field generation unit.

In this case, the magnetic field generation unit may be able to apply a rotating magnetic field.

Moreover, a direction of the magnetic field applied by the magnetic field generation unit and a direction of the light emitted from the light source may be directions within the same plane.

Moreover, the detection unit may further detect whether or not the target biomaterial exists based on a quantity of the transmitted light after the magnetic field generation unit stops applying the magnetic field.

Moreover, the detection unit may further detect whether or not the target biomaterial exists by using a calibration curve that is created in advance with the use of a known sample.

Furthermore, a biosensor of the present invention, which uses magnetic microparticles, may comprise: a magnetic field generation unit that is able to apply a magnetic field, a direction of which changes at least in two directions, to a dispersion liquid that contains magnetic microparticles to which a target biomaterial can bind; a light source that emits light of a predetermined wavelength to the dispersion liquid; a light receiving unit that receives a light reflected from the dispersion liquid; and a detection unit that detects whether or not the target biomaterial exists based on the amount of change in the quantity of the reflected light received by the light receiving unit, which is caused by a direction change of the magnetic field applied by the magnetic field generation unit.

Furthermore, the biosensor may include an optical waveguide that leads the light from the light source to the dispersion liquid.

Moreover, the light receiving unit may be disposed on a dispersion liquid-side tip of the optical waveguide.

### Advantages of the Invention

The advantage is that the biosensor of the present invention that uses the magnetic microparticles is able to detect a biomaterial with higher levels of sensitivity.

### Brief Description of the Drawings

FIG. 1 is a schematic perspective view illustrating the configuration of a biosensor according to a first embodiment of the present invention.
FIG. 2 is a configuration block diagram of a biosensor according to the first embodiment of the present invention.
FIG. 3 is a top view illustrating magnetic field generation units that can apply a rotating magnetic field to a dispersion liquid.
FIG. 4 is a side view illustrating another way of disposing a magnetic field generation unit in the biosensor according to the first embodiment of the present invention.
FIG. 5 is a schematic perspective view illustrating changes of magnetic microparticles contained in the dispersion liquid of the biosensor according to the first embodiment of the present invention.
FIG. 6 is a graph illustrating the differences in the amount of change in the quantity of the transmitted light, which occur depending on whether or not a target biomaterial exists, in the biosensor according to the first embodiment of the present invention.
FIG. 7 is a graph illustrating the differences in the amount of change in the quantity of the transmitted light, which occur depending on whether or not a target biomaterial exists under other conditions, in the biosensor according to the first embodiment of the present invention.
FIG. 8 is a schematic perspective view illustrating the configuration of a biosensor according to a second embodiment of the present invention.
FIG. 9 is a schematic perspective view illustrating the other configuration of a biosensor according to the second embodiment of the present invention.

### Best Mode for Carrying Out the Invention

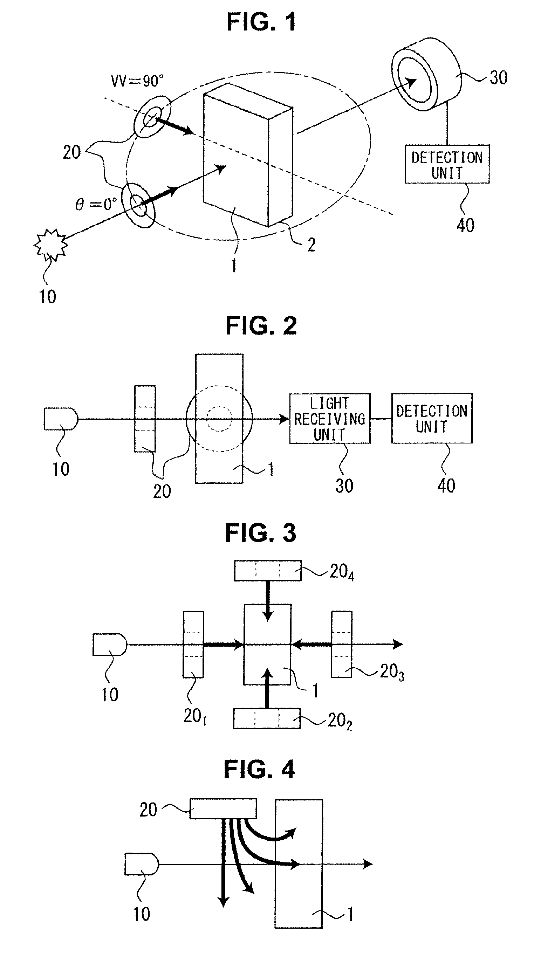

Preferred embodiments of the present invention will be described below with reference to the accompanying drawings. FIG. 1 is a schematic perspective view illustrating the configuration of a biosensor according to a first embodiment of the present invention. FIG. 2 is a configuration block diagram of a biosensor according to the first embodiment of the present invention. As shown in the drawings, a biosensor of the first embodiment of the present invention mainly includes a light source 10, magnetic field generation units 20, a light receiving unit 30, and a detection unit 40. In addition, an optical system including a collimator lens and a condensing lens is appropriately used when necessary.

A dispersion liquid 1, which is an object to be measured by the biosensor of the present invention, contains magnetic microparticles, which a target biomaterial can bind to. The dispersion liquid 1 is stored in an optically transparent container 2. In addition, the term "optically transparent" as used in the present specification means allowing the light from the light source 10 to pass therethrough in an excellent manner in a wavelength region used by an optical system that is used to detect a biomaterial; and does not necessarily mean having transparency in the entire wavelength region. The target biomaterial is a so-called antigen, which is for example made up of an oligosaccharide, a peptide and the like.

Surface treatment is applied to the magnetic microparticles contained in the dispersion liquid 1 so that the biomaterial can bind to. All that is required is for a material that binds specifically to the biomaterial, such as an antibody, to be provided on the surfaces of the magnetic microparticles. For example, to the surfaces of the magnetic microparticles, a probe consisting of a sulfur atom and a thiol group is fixed; an oligosaccharide, which turns out to be a target biomaterial to which biotin is attached, is so structured as to bind to the probe. In addition, as to the magnetic microparticles contained in the dispersion liquid 1, the surface treatment is not necessarily applied in such a way that the biomaterial can bind to all the magnetic microparticles. The magnetic microparticles are not specifically restricted. However, it is preferred that the particle diameters of the magnetic microparticles be 100 nm or less, about a size equivalent to unimolecular DNA, for example. Such a size reduces the difference in size between the magnetic microparticles and the biomaterial, making it easier for the magnetic microparticles and the biomaterial to bind to each other. The magnetic microparticles may be ferromagnetic or paramagnetic, and may be ferrite, alnico or the like. More specifically, the magnetic microparticles made of various magnetic materials, including the following, are available: FePt, Co, Ni, Fe, MnSb, and MnAs. In addition, it is possible to use commercially available magnetic microparticles. For example, it is possible to use magnetic microparticles with a particle diameter of 1 µm, such as Dynabeads MyOne Streptavidin manufactured by Invitrogen Corporation.

To the dispersion liquid 1 containing a plurality of magnetic microparticles, a magnetic field is applied by a magnet, coil or the like. Then, along a direction in which the magnetic field is applied, a plurality of magnetic microparticles are adsorbed in the shape of a column, and a plurality of magnetic microparticle chains are formed as a result. Such a phenomenon is described, for example, "Field-induced Structures in Ferrofluid Emulsions", Jing Liu et al., Physical Review Letters, Vol. 74, No. 14, April 3, 1995.

The following describes each of the components of the biosensor according to the first embodiment of the present invention. The light source 10 is designed to emit a predetermined wavelength of light to the dispersion liquid 1. As to the light source 10, a source capable of emitting infrared light may be used, for example. Moreover, a white light source, a filter, and the like may be used in combination.

The magnetic field generation units 20 are designed to apply a magnetic field, a direction of which changes at least in two directions, to the dispersion liquid 1. As in the example shown in the drawing, the directions of the applied magnetic field may for example be a 0-degree direction and a 90-degree direction, respectively, when the direction in which the light is emitted from the light source 10 is θ = 0 degree. For example, when the magnetic field generation units 20 are made from two electromagnets, the electromagnets are so controlled as to be turned ON and OFF alternately. As a result, it is possible to alternately apply a 0-degree and a 90-degree magnetic field to the dispersion liquid 1.

The magnetic field generation units 20 may be able to apply a rotating magnetic field to the dispersion liquid 1. FIG. 3 is a top view illustrating magnetic field generation units that can apply a rotating magnetic field to the dispersion liquid. As shown in the drawing, the dispersion liquid 1 is positioned at the center, and electromagnets 20₁ to 20₄ are disposed every 90 degrees so as to face each other. The electromagnets 20₁ to 20₄ are so structured as to alternately apply a magnetic field in four directions. As a result, a rotating magnetic field is applied to the dispersion liquid 1. In addition, as to the rotating speed of the rotating magnetic field, the rotating magnetic field may be appropriately rotated at a speed that does not divide the magnetic microparticle chains.

Moreover, as to the magnetic field generation units 20, it is preferred that the direction of the applied magnetic field and the direction of the light emitted from the light source 10 be directions within the same plane. For example, as shown in FIGS. 1 and 2 and other drawings, in the case of 0 degree, the light may be emitted to the dispersion liquid 1 from a hole at a central portion of an electromagnet of the magnetic field generation unit 20. As shown in FIG. 4, which is a side view illustrating another way of disposing a magnetic field generation unit, the magnetic field generation unit 20 may be so disposed that the axis of a magnet of the magnetic field generation unit 20 is perpendicular to an optical axis of the light source 10. Because the arrangement and position of the magnetic field generation unit 20 are adjusted as described above, the structure is possible that enables a magnetic flux that extends laterally to be applied into the same plane as the optical axis of the light source 10 relative to the dispersion liquid 1. Since the direction of the applied magnetic field and the direction of the light emitted from the light source are within the same side surface, the amount of change of the transmitted light, which will be described later, becomes larger.

The intensity of the magnetic field of the magnetic field generation unit 20 varies according to magnetic properties of the magnetic microparticles, such as magnetic moment. However, the intensity may be set appropriately by taking into account the following: the size of a magnetic microparticle chain that can be formed, and the magnetic field intensity at which the magnetic microparticles do not precipitate. For example, the applied magnetic field may be up to about 71.6 kA/m (about 900 Oe). Even with a smaller magnetic field, a magnetic microparticle column can be formed.

The light receiving unit 30 is designed to receive the transmitted light from the dispersion liquid 1. The light receiving unit 30 may be so formed as to include a photodiode and the like. The light receiving unit 30 may be so formed as to receive only a light beam of an optical-axis direction from the light source 10 out of the transmitted light from the dispersion liquid; or alternatively, the light receiving unit 30 may be so formed as to receive the scattered light emitted from the dispersion liquid.

The detection unit 40 is designed to detect the amount of change in the quantity of the transmitted light received by the light receiving unit 30, and detect whether or not a target biomaterial exists based on the detection results. Hereinafter, the amount of change in the quantity of the transmitted light detected by the detection unit 40 will be described in detail.

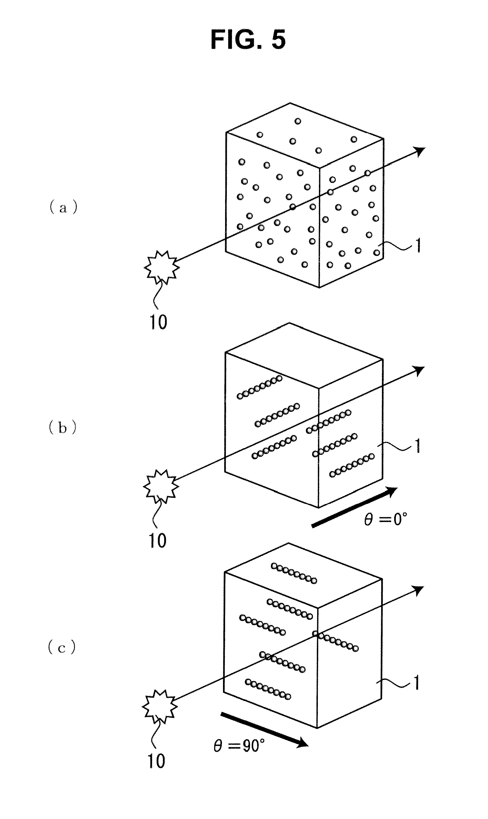

FIG. 5 is a schematic perspective view illustrating changes of magnetic microparticles contained in the dispersion liquid 1. FIG. 5(a) shows the situation where no magnetic field is applied. FIG. 5(b) shows the case where a magnetic field is applied in a 0-degree direction. FIG. 5(c) shows the case where a magnetic field is applied in a 90-degree direction. In addition, there is some exaggeration in the drawings as to the size of the magnetic microparticles in a way that makes clear the motion of the magnetic microparticles.

As shown in FIG. 5(a), before a magnetic field is applied to the dispersion liquid 1, the magnetic microparticles are dispersed in the dispersion liquid 1. As a magnetic field is applied, as shown in FIGS. 5(b) and 5(c), a plurality of magnetic microparticles are adsorbed in the shape of a column along the direction of the applied magnetic field, and a plurality of magnetic microparticle chains are formed. At this time, the lengths of the magnetic microparticle chains vary depending on whether or not a biomaterial exists in the dispersion liquid 1. If a biomaterial exists in the dispersion liquid 1, the biomaterial binds to the antibody of a magnetic microparticle. Furthermore, to the biomaterial that becomes bound to the magnetic microparticle, another magnetic microparticle could bind. Therefore, if there is a biomaterial, the magnetic microparticle chains become longer in length. The differences in length affect the amount of change in the quantity of the transmitted light that has passed through the dispersion liquid 1 from the light source 10 at a time when the direction of the magnetic field is changed. Therefore, by measuring the amount of change, it is possible to detect whether or not a target biomaterial exists.

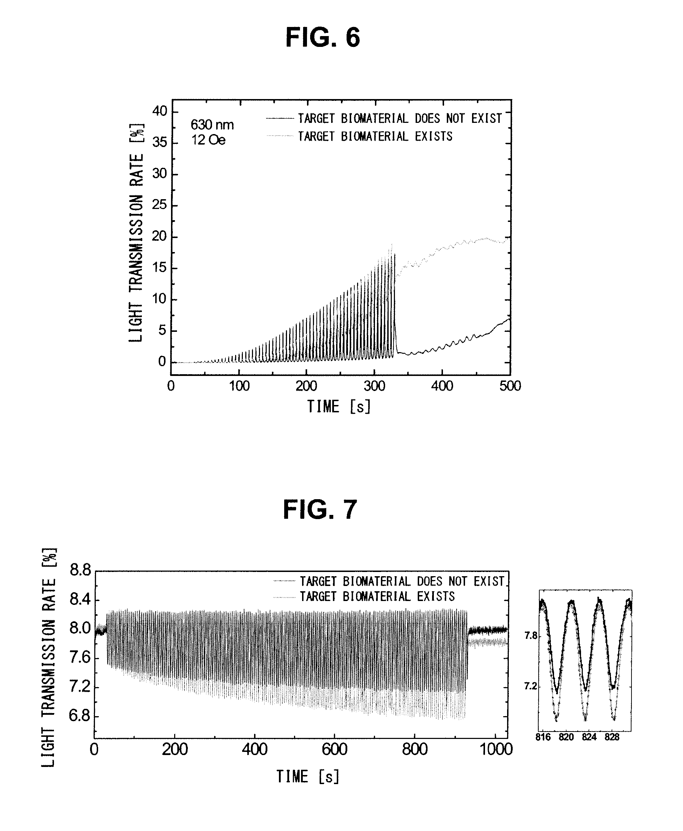

FIG. 6 shows a graph illustrating the differences in the amount of change in the quantity of the transmitted light, which occur depending on whether or not a target biomaterial exists. The drawing shows changes in the quantity of the transmitted light received by the light receiving unit at a time when a rotating magnetic field was applied to the dispersion liquid. The horizontal axis represents time. In addition, the light source used was able to emit light with a wavelength of 630 nm; the magnetic microparticles used were 130 nm in particle diameter. For the target biomaterial, avidin was used. FIG. 6 shows the quantity of the transmitted light at a time when a rotating magnetic field was applied to a dispersion liquid from magnetic field generation units under the above conditions. In addition, the intensity of the rotating magnetic field was about 955 A/m (12 Oe); the rotational frequency was 0.1 Hz. For the light receiving unit, USB2000, manufactured by Ocean Optics, Inc., was used.

The transmitted light quantity was measured under the above conditions. As shown in the drawing, there were changes in the amount of change in the quantity of the transmitted light between when the target biomaterial existed and when the target biomaterial did not exist. More specifically, under the above-noted conditions, the amount of change in the quantity of the transmitted light was smaller when the target biomaterial existed than when the target biomaterial did not. Accordingly, the detection unit is able to detect whether or not the target biomaterial exists by detecting the amount of change (amplitude) in the quantity of the transmitted light received.

Furthermore, it is clear from what is shown in the drawing that, after the application of the magnetic field from the magnetic field generation units was stopped (after about 325 seconds), the differences in the quantity of the transmitted light became larger compared with the time when the magnetic field was not yet applied. That is, the transmitted light quantity (level) became larger when the target biomaterial existed than when the target biomaterial did not, suggesting that the magnetic microparticles became bound to each other due to the existence of the target biomaterial even after the application of the magnetic field was stopped. Accordingly, the detection unit is also able to detect whether or not the target biomaterial exists by detecting the quantity (level) of the transmitted light quantity.

As to the detection process by the detection unit, a difference of detection results before and after the introduction of a sample may be used to detect whether or not the target biomaterial exists; or alternatively, a calibration curve may be created in advance with the use of a known sample so that the calibration curve can be used to detect whether or not the target biomaterial of an unknown sample exists.

In addition, as to the amount of change (amplitude) of the transmitted light quantity and the volume (level) thereof, depending on the particle diameter of the magnetic microparticles and the frequency of the light from the light source or the performance of the light receiving unit (grating or the like), the most appropriate conditions vary. Depending on the conditions, the change characteristics vary. FIG. 7 shows a graph illustrating the differences in the amount of change in the quantity of the transmitted light, which occur depending on whether or not a target biomaterial exists under other conditions. The drawing shows the changes in the transmitted light quantity at a time when the light source used was able to emit light with a wavelength of 785 nm, and when the particle diameter of the magnetic microparticles used was 250 nm. In addition, for the light receiving unit, USB2000, manufactured by Ocean Optics, Inc., was similarly used. Under the conditions, the amount of change in the quantity of the transmitted light was larger when the target biomaterial existed than when the target biomaterial did not. The transmitted light quantity was smaller when the target biomaterial existed than when the target biomaterial did not.

In that manner, when the biosensor of the first embodiment of the present invention changes the direction of a magnetic field when applying the magnetic field to the dispersion liquid under predetermined conditions, the amount of change in the quantity of the transmitted light is caused by a direction change of the magnetic field. Therefore, using the amount of change described above, it is possible to detect the existence of the target biomaterial. The length of a magnetic microparticle chain formed becomes longer as the concentration of the target biomaterial rises. Therefore, it is possible to detect not only whether or not the target biomaterial exists but also the concentration thereof.

Moreover, the biosensor of the first embodiment of the present invention changes the direction of the magnetic field, causing the magnetic microparticle chains to be shaken. Therefore, there is also an effect of stirring the dispersion liquid. As a result, compared with the case where a magnetic field is simply applied in one direction, it is also possible to ensure that the target biomaterial easily binds to the magnetic microparticles.

Furthermore, as shown in FIGS. 6 and 7, according to the biosensor of the first embodiment of the present invention, the differences in the amount of change in the quantity of the transmitted light, which were associated with whether or not the target biomaterial existed, appeared immediately after the rotating magnetic field was applied. It was clear that the trend of the amount of change thereof remained unchanged over time during the process of applying the magnetic field. The reason was that, particularly when the rotating magnetic field was applied, the magnetic microparticle chains positioned between the electromagnets did not precipitate and remained oriented to the direction of the applied magnetic field. As a result, the measurement results were not affected by precipitation even as time went by. As described above as to the prior art, for example, in the case of Patent Document 1, since the magnetic microparticles precipitate with time, the concentration also changes. According to the present invention, because the measurement takes place during the process of applying the magnetic field, it is possible to solve the above problem.

The magnetic field generation units are not limited to those described above, which apply a rotating magnetic field. As to the direction of the magnetic field applied by the magnetic field generation units, all that is required is for the magnetic field to change at least in two directions so that the amount of change in the quantity of the transmitted light can be measured. In addition, the two directions, 0 and 90 degrees, realize the situation where the largest amount of change can be measured. As described above, the differences in the amount of change in the quantity of the transmitted light appeared immediately after the magnetic field was applied. Therefore, it is sufficient for the biosensor of the present invention to measure the transmitted light quantity at least in the following two situations: the transmitted light quantity at a time when the magnetic field is applied in one direction, and the transmitted light quantity at a time when the magnetic field is applied in another one direction. Therefore, it takes a very short period of time to detect whether or not the target biomaterial exists.

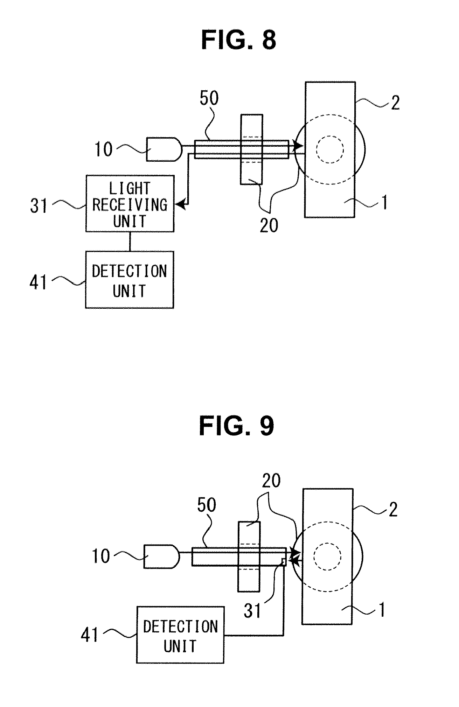

The following describes a biosensor according to a second embodiment of the present invention. The biosensor of the first embodiment uses the transmitted light quantity to detect whether or not the target biomaterial exists. The biosensor of the second embodiment is designed to receive the light reflected from the dispersion liquid, and uses the quantity of the reflected light to detect whether or not the target biomaterial exists. FIG. 8 is a schematic perspective view illustrating the configuration of a biosensor according to the second embodiment of the present invention. In the drawing, the portions indicated by the same reference symbols as those in FIG. 1 represent substantially the same components. Therefore, the detailed description of the components will be omitted.

As shown in the drawing, in the biosensor of the second embodiment, the light emitted from the light source 10 enters the dispersion liquid 1. The biosensor includes a light receiving unit 31 that receives the light reflected from the dispersion liquid. A detection unit 41 detects whether or not the target biomaterial exists based on the amount of change in the quantity of the reflected light received by the light receiving unit 31. As in the case of the transmitted light quantity described in the first embodiment, the quantity of the reflected light is caused by a direction change of a magnetic field applied by the magnetic field generation units 20. As to change characteristics, the reaction is opposite to that of the transmitted light quantity. Accordingly, even when the reflected light quantity is used, as in the case where the transmitted light quantity is used, it is possible to detect whether or not the target biomaterial exists.

In the example shown in the drawing, the light from the light source 10 is emitted to the dispersion liquid 1 using an optical waveguide 50, such as optical fiber. The structure here allows the reflected light to enter the light receiving unit 31 via the optical waveguide 50. For example, all that is required for the above is as follows: the optical waveguide 50 is formed by bundling a plurality of optical fibers, one central fiber of the bundle is connected to the light receiving unit 31, and the other peripheral optical fibers are connected to the light source 10. Among the specific products that have the above configuration is R400-7-VIS-NIR, manufactured by Ocean Optics, Inc. As a result, it is possible to receive the regular reflection of light on a surface of a tip section of the optical waveguide 50, or the diffused reflection. Moreover, as shown in FIG. 9, the light receiving unit 31 may be so formed that a photoelectric conversion device, such as CCD, is directly disposed on a dispersion liquid-side tip of the optical waveguide 50.

As described above, according to the biosensor of the present invention, it is possible to detect whether or not the target biomaterial exists with high levels of sensitivity. That is, if a sensor is designed to measure the magnetism of magnetic microparticles and therefore detect whether or not a target biomaterial exists in the same way as a conventional technique, it could not be possible to detect because of the insufficient sensitivity of the magnetic sensor at a time when the magnetic microparticles are small in diameter and when the magnetism is extremely small. However, the biosensor of the present invention is not intended to measure the magnetism. Based on change in the transmitted or reflected light, the biosensor of the present invention is able to detect whether or not the target biomaterial exists. As a result, compared with a conventional technique, higher-sensitivity measurement is possible.

In addition, the biosensor of the present invention that uses the magnetic microparticles is not limited to those in the above embodiments shown in the drawings. Needless to say, various changes may be made without departing from the subject-matter of the present invention. The measurement conditions, the measurement results, and the like are only one specific embodiment among many and the present invention is not limited thereto.

### Explanation of Reference Symbols

- 1:: Dispersion liquid
- 2:: Container
- 10:: Light source
- 20:: Magnetic field generation unit
- 30, 31:: Light receiving unit
- 40, 41:: Detection unit
- 50:: Optical waveguide

## Claims

1. A biosensor that uses magnetic microparticles, the biosensor comprising:
a light source that emits light of a predetermined wavelength to a dispersion liquid that contains magnetic microparticles to which a target biomaterial can bind;
a magnetic field generation unit that is able to apply a magnetic field, a direction of which changes at least in two directions, to the dispersion liquid;
a light receiving unit that receives a transmitted light from the dispersion liquid; and
a detection unit that detects whether or not the target biomaterial exists based on an amount of change in a quantity of the transmitted light received by the light receiving unit, which is caused by a direction change of the magnetic field applied by the magnetic field generation unit.

2. The biosensor according to claim 1, in which the magnetic field generation unit is able to apply a rotating magnetic field.

3. The biosensor according to claim 1 or 2, in which a direction of the magnetic field applied by the magnetic field generation unit and a direction of the light emitted from the light source are directions within the same plane.

4. The biosensor according to any one of claims 1 to 3, in which the detection unit further detects whether or not the target biomaterial exists based on a quantity of the transmitted light after the magnetic field generation unit stops applying the magnetic field.

5. The biosensor according to any one of claims 1 to 4, in which the detection unit further detects whether or not the target biomaterial exists by using a calibration curve that is created in advance with the use of a known sample.

6. A biosensor that uses magnetic microparticles, the biosensor comprising:
a magnetic field generation unit that is able to apply a magnetic field, a direction of which changes at least in two directions, to a dispersion liquid that contains magnetic microparticles to which a target biomaterial can bind;
a light source that emits light of a predetermined wavelength to the dispersion liquid;
a light receiving unit that receives a light reflected from the dispersion liquid; and
a detection unit that detects whether or not the target biomaterial exists based on the amount of change in the quantity of the reflected light received by the light receiving unit, which is caused by a direction change of the magnetic field applied by the magnetic field generation unit.

7. The biosensor according to claim 6, which further comprising an optical waveguide that leads the light from the light source to the dispersion liquid.

8. The biosensor according to claim 7, in which the light receiving unit is disposed on a dispersion liquid-side tip of the optical waveguide.
